# EUROPEAN PATENT SPECIFICATION

(11) **EP 2 157 966 B1**
(45) Date of publication and mention of the grant of the patent: **13.03.2019**
(21) Application number: 08760698.4
(22) Date of filing: 06.06.2008
(51) Int. Cl.: A61K 8/97, A61Q 19/08, A61K 36/28

(54) **MC-1R, MC-2R, AND/OR µ OPIOID RECEPTORS STIMULATION**
MC-1R-, MC-2R- UND/ODER µ-OPIOID-REZEPTOR-STIMULATION
STIMULATION DE MC-1R, MC-2R ET/OU DES RÉCEPTEURS µ-OPIOÏDES

(30) Priority: 06.06.2007 FR 0755529
(43) Date of publication of application: 03.03.2010
(73) Proprietor: BASF Beauty Care Solutions France SAS, 69007 Lyon (FR)
(72) Inventor: PAIN, Sabine, 38200 Vienne (FR); DEZUTTER, Colette, F-38200 Vienne (FR); ANDRE, Valérie, F-69420 Ampuis (FR); REYMERMIER, Corinne, F-69390 Charly (FR); ORLY, Isabelle, F-69540 Irigny (FR); PERRIER, Eric, F-38138 Les Cotes D'arey (FR)
(74) Representative: Mendelsohn, Isabelle M. N.
(86) International application number: PCT/EP2008/057129
(87) International publication number: WO 2008/148891

(56) References cited:
- EP-A- 1 543 825
- WO-A-2007/030666
- CH-A5- 650 147
- DE-U1- 20 112 636
- FR-A- 2 578 422
- KR-A- 20040 046 507
- US-A- 3 522 350
- US-A1- 2004 214 851

### SEQUENCE LISTING

<110> BASF BEAUTY CARE SOLUTIONS FRANCE SAS
<120> MC-1R, MC-2R, and µ opioid receptors stimulation
<130> H197810/68 PCT1
<150> FR 07 55529
   <151> 2007-06-06
<160> 14
<170> PatentIn version 3.3
<210> 1
   <211> 2360
   <212> DNA
   <213> Homo sapiens
<400> 1
   gagagggcag gtcccgggga agctccggac tcctagaggg gcggccaggt gggggccctg      60
   gtgaccagga cagactgtgg tgttttttaa cgtaaaggag atccgcggtg tgagggaccc     120
   cctgggtcct gcacgccgcc tggtggcagg ccgggccatg gtgggtgctc acgcccccgg    180
   catgtggccg ccctcagtgg gaggggctct gagaacgact ttttaaaacg cagagaaaag    240
   ctccattctt cccaggacct cagcgcagcc ctggcccagg aaggcaggag acagaggcca    300
   ggacggtcca gaggtgtcga aatgtcctgg ggacctgagc agcagccacc agggaagagg    360
   cagggaggga gctgaggacc aggcttggtt gtgagaatcc ctgagcccag gcggtagatg    420
   ccaggaggtg tctggactgg ctgggccatg cctgggctga cctgtccagc cagggagagg    480
   gtgtgagggc agatctgggg gtgcccagat ggaaggaggc aggcatgggg gacacccaag    540
   gccccctggc agcaccatga actaagcagg acacctggag gggaagaact gtggggacct    600
   ggaggcctcc aacgactcct tcctgcttcc tggacaggac tatggctgtg cagggatccc    660
   agagaagact tctgggctcc ctcaactcca cccccacagc catcccccag ctggggctgg    720
   ctgccaacca gacaggagcc cggtgcctgg aggtgtccat ctctgacggg ctcttcctca    780
   gcctggggct ggtgagcttg gtggagaacg cgctggtggt ggccaccatc gccaagaacc    840
   ggaacctgca ctcacccatg tactgcttca tctgctgcct ggccttgtcg gacctgctgg    900
   tgagcgggag caacgtgctg gagacggccg tcatcctcct gctggaggcc ggtgcactgg    960
   tggcccgggc tgcggtgctg cagcagctgg acaatgtcat tgacgtgatc acctgcagct   1020
   ccatgctgtc cagcctctgc ttcctgggcg ccatcgccgt ggaccgctac atctccatct   1080
   tctacgcact gcgctaccac agcatcgtga ccctgccgcg ggcgcggcga gccgttgcgg   1140
   ccatctgggt ggccagtgtc gtcttcagca cgctcttcat cgcctactac gaccacgtgg   1200
   ccgtcctgct gtgcctcgtg gtcttcttcc tggctatgct ggtgctcatg gccgtgctgt    1260
   acgtccacat gctggcccgg gcctgccagc acgcccaggg catcgcccgg ctccacaaga    1320
   ggcagcgccc ggtccaccag ggctttggcc ttaaaggcgc tgtcaccctc accatcctgc    1380
   tgggcatttt cttcctctgc tggggcccct tcttcctgca tctcacactc atcgtcctct    1440
   gccccgagca ccccacgtgc ggctgcatct tcaagaactt caacctcttt ctcgccctca    1500
   tcatctgcaa tgccatcatc gaccccctca tctacgcctt ccacagccag gagctccgca    1560
   ggacgctcaa ggaggtgctg acatgctcct ggtgagcgcg gtgcacgcgg ctttaagtgt    1620
   gctgggcaga gggaggtggt gatattgtgt ggtctggttc ctgtgtgacc ctgggcagtt    1680
   ccttacctcc ctggtccccg tttgtcaaag aggatggact aaatgatctc tgaaagtgtt    1740
   gaagcgcgga cccttctggg tccagggagg ggtccctgca aaactccagg caggacttct    1800
   caccagcagt cgtggggaac ggaggaggac atggggaggt tgtggggcct caggctccgg    1860
   gcaccagggg ccaacctcag gctcctaaag agacattttc cgcccactcc tgggacactc    1920
   cgtctgctcc aatgactgag cagcatccac cccaccccat ctttgctgcc agctctcagg    1980
   accgtgccct cgtcagctgg gatgtgaagt ctctgggtgg aagtgtgtgc caagagctac    2040
   tcccacagca gccccaggag aaggggcttt gtgaccagaa agcttcatcc acagccttgc    2100
   agcggctcct gcaaaaggag gtgaaatccc tgcctcaggc caagggacca ggtttgcagg    2160
   agccccccta gtggtatggg gctgagccct cctgagggcc ggttctaagg ctcagactgg    2220
   gcactggggc ctcagcctgc tttcctgcag cagtcgccca agcagacagc cctggcaaat    2280
   gcctgactca gtgaccagtg cctgtgagca tggggccagg aaagtctggt aataaatgtg    2340
   actcagcatc acccacctta                                                2360
<210> 2
   <211> 894
   <212> DNA
   <213> Homo sapiens
<400> 2
   atgaagcaca ttatcaactc gtatgaaaac atcaacaaca cagcaagaaa taattccgac     60
   tgtcctcgtg tggttttgcc ggaggagata tttttcacaa tttccattgt tggagttttg    120
   gagaatctga tcgtcctgct ggctgtgttc aagaataaga atctccaggc acccatgtac    180
   tttttcatct gtagcttggc catatctgat atgctgggca gcctatataa gatcttggaa    240
   aatatcctga tcatattgag aaacatgggc tatctcaagc cacgtggcag ttttgaaacc    300
   acagccgatg acatcatcga ctccctgttt gtcctctccc tgcttggctc catcttcagc    360
   ctgtctgtga ttgctgcgga ccgctacatc accatcttcc acgcactgcg gtaccacagc    420
   atcgtgacca tgcgccgcac tgtggtggtg cttacggtca tctggacgtt ctgcacgggg    480
   actggcatca ccatggtgat cttctcccat catgtgccca cagtgatcac cttcacgtcg    540
   ctgttcccgc tgatgctggt cttcatcctg tgcctctatg tgcacatgtt cctgctggct    600
   cgatcccaca ccaggaagat ctccaccctc cccagagcca acatgaaagg ggccatcaca    660
   ctgaccatcc tgctcggggt cttcatcttc tgctgggccc cctttgtgct tcatgtcctc    720
   ttgatgacat tctgcccaag taacccctac tgcgcctgct acatgtctct cttccaggtg    780
   aacggcatgt tgatcatgtg caatgccgtc attgacccct tcatatatgc cttccggagc    840
   ccagagctca gggacgcatt caaaaagatg atcttctgca gcaggtactg gtag          894
<210> 3
   <211> 1891
   <212> DNA
   <213> homo sapiens
<400> 3
   gatgagcctc tgtgaactac taaggtggga gggggctata cgcagaggag aatgtcagat     60
   gctcagctcg gtcccctccg cctgacgctc ctctctgtct cagccaggac tggtttctgt    120
   aagaaacagc aggagctgtg gcagcggcga aaggaagcgg ctgaggcgct tggaacccga    180
   aaagtctcgg tgctcctggc tacctcgcac agcggtgccc gcccggccgt cagtaccatg    240
   gacagcagcg ctgcccccac gaacgccagc aattgcactg atgccttggc gtactcaagt    300
   tgctccccag cacccagccc cggttcctgg gtcaacttgt cccacttaga tggcaacctg    360
   tccgacccat gcggtccgaa ccgcaccgac ctgggcggga gagacagcct gtgccctccg    420
   accggcagtc cctccatgat cacggccatc acgatcatgg ccctctactc catcgtgtgc    480
   gtggtggggc tcttcggaaa cttcctggtc atgtatgtga ttgtcagata caccaagatg    540
   aagactgcca ccaacatcta cattttcaac cttgctctgg cagatgcctt agccaccagt    600
   accctgccct tccagagtgt gaattaccta atgggaacat ggccatttgg aaccatcctt    660
   tgcaagatag tgatctccat agattactat aacatgttca ccagcatatt caccctctgc    720
   accatgagtg ttgatcgata cattgcagtc tgccaccctg tcaaggcctt agatttccgt    780
   actccccgaa atgccaaaat tatcaatgtc tgcaactgga tcctctcttc agccattggt    840
   cttcctgtaa tgttcatggc tacaacaaaa tacaggcaag gttccataga ttgtacacta    900
   acattctctc atccaacctg gtactgggaa aacctgctga agatctgtgt tttcatcttc    960
   gccttcatta tgccagtgct catcattacc gtgtgctatg gactgatgat cttgcgcctc   1020
   aagagtgtcc gcatgctctc tggctccaaa gaaaaggaca ggaatcttcg aaggatcacc   1080
   aggatggtgc tggtggtggt ggctgtgttc atcgtctgct ggactcccat tcacatttac   1140
   gtcatcatta aagccttggt tacaatccca gaaactacgt tccagactgt ttcttggcac   1200
   ttctgcattg ctctaggtta cacaaacagc tgcctcaacc cagtccttta tgcatttctg   1260
   gatgaaaact tcaaacgatg cttcagagag ttctgtatcc caacctcttc caacattgag   1320
   caacaaaact ccactcgaat tcgtcagaac actagagacc acccctccac ggccaataca   1380
   gtggatagaa ctaatcatca gctagaaaat ctggaagcag aaactgctcc gttgccctaa   1440
   cagggtctca tgccattccg accttcacca agcttagaag ccaccatgta tgtggaagca   1500
   ggttgcttca agaatgtgta ggaggctcta attctctagg aaagtgcctg cttttaggtc   1560
   atccaacctc tttcctctct ggccactctg ctctgcacat tagagggaca gccaaaagta   1620
   agtggagcat ttggaaggaa aggaatatac cacaccgagg agtccagttt gtgcaagaca   1680
   cccagtggaa ccaaaaccca tcgtggtatg tgaattgaag tcatcataaa aggtgaccct   1740
   tctgtctgta agattttatt ttcaagcaaa tatttatgac ctcaacaaag aagaaccatc   1800
   ttttgttaag ttcaccgtag taacacataa agtaaatgct acctctgatc aaagcacctt   1860
   gaatggaagg tccgagtctt tttagtgttt t                                  1891
<210> 4
   <211> 1071
   <212> DNA
   <213> homo sapiens
<400> 4
   agcggcggcg aaggagggga agaagagccg cgaccgagag aggccgccga gcgtccccgc     60
   cctcagagag cagcctcccg agacagagcc tcagcctgcc tggaagatgc cgagatcgtg    120
   ctgcagccgc tcgggggccc tgttgctggc cttgctgctt caggcctcca tggaagtgcg    180
   tggctggtgc ctggagagca gccagtgtca ggacctcacc acggaaagca acctgctgga    240
   gtgcatccgg gcctgcaagc ccgacctctc ggccgagact cccatgttcc cgggaaatgg    300
   cgacgagcag cctctgaccg agaacccccg gaagtacgtc atgggccact tccgctggga    360
   ccgattcggc cgccgcaaca gcagcagcag cggcagcagc ggcgcagggc agaagcgcga    420
   ggacgtctca gcgggcgaag actgcggccc gctgcctgag ggcggccccg agccccgcag    480
   cgatggtgcc aagccgggcc cgcgcgaggg caagcgctcc tactccatgg agcacttccg    540
   ctggggcaag ccggtgggca agaagcggcg cccagtgaag gtgtacccta acggcgccga    600
   ggacgagtcg gccgaggcct tccccctgga gttcaagagg gagctgactg gccagcgact    660
   ccgggaggga gatggccccg acggccctgc cgatgacggc gcaggggccc aggccgacct    720
   ggagcacagc ctgctggtgg cggccgagaa gaaggacgag ggcccctaca ggatggagca    780
   cttccgctgg ggcagcccgc ccaaggacaa gcgctacggc ggtttcatga cctccgagaa    840
   gagccagacg cccctggtga cgctgttcaa aaacgccatc atcaagaacg cctacaagaa    900
   gggcgagtga gggcacagcg ggccccaggg ctaccctccc ccaggaggtc gaccccaaag    960
   ccccttgctc tcccctgccc tgctgccgcc tcccagcctg gggggtcgtg gcagataatc   1020
   agcctcttaa agctgcctgt agttaggaaa taaaaccttt caaatttcac a            1071
<210> 5
   <211> 20
   <212> DNA
   <213> artificial sequence
<220>
   <223> sonde Actine Sens
<400> 5
   gtggggcgcc ccaggcacca 20
<210> 6
   <211> 24
   <212> DNA
   <213> artificial sequence
<220>
   <223> sonde Actine Antisens
<400> 6
   ctccttaatg tcacgcacga tttc 24
<210> 7
   <211> 20
   <212> DNA
   <213> artificial sequence
<220>
   <223> Sonde MCR sens
<400> 7
   gggctctgag aacgactttt 20
<210> 8
   <211> 20
   <212> DNA
   <213> artificial sequence
<220>
   <223> sonde MCR1 Antisens
<400> 8
   ccgggctcct gtctggttgg 20
<210> 9
   <211> 22
   <212> DNA
   <213> artificial sequence
<220>
   <223> sonde MCR2 Sens
<400> 9
   tcacgtcgct gttcccgctg at 22
<210> 10
   <211> 26
   <212> DNA
   <213> artificial sequence
<220>
   <223> sonde MCR2 Antisens
<400> 10
   aagagagaca tgtagcaggc gcagta 26
<210> 11
   <211> 26
   <212> DNA
   <213> artificial sequence
<220>
   <223> sonde µ opioide Sens
<400> 11
   ctcagccagg actggtttct gtaaga 26
<210> 12
   <211> 23
   <212> DNA
   <213> artificial sequence
<220>
   <223> sonde µ opioide Antisens
<400> 12
   tcggacaggt tgccatctaa gtg 23
<210> 13
   <211> 20
   <212> DNA
   <213> artificial sequence
<220>
   <223> sonde POMC Sens
<400> 13
   cgcccagtga aggtgtaccc 20
<210> 14
   <211> 24
   <212> DNA
   <213> artificial sequence
<220>
   <223> sonde POMC Antisens
<400> 14
   ggcgtctggc tcttctcgga ggtc 24

## Description

The invention concerns the active components stimulating the expression of neuromediating receptors coded by the POMC (proopiomelanocortin) gene and possibly stimulating the expression of corresponding neuromediators. The invention also addresses the use of a composition containing at least one substance active in the expression of neuromediating receptors coded by the POMC gene at the cutaneous level.

### Current understanding of the subject:

Due to its sensory functions, the skin is a primordial source of information for the individual. Peripheral nerves are responsible for the innervation of the skin and they possess axons whose cellular bodies are located along the spinal cord. Cutaneous innervation includes, among others, sensory nerves and autonomous sympathetic fibers. Superficial free nerves endings are the only sensory fibers that penetrate to the interior of the epidermis. An important exchange exists between skin cells and cutaneous nerves. Skin cells synthesize numerous neuromediators that act on the skin neurons (paracrine route). Cutaneous cells also possess receptors corresponding to certain neuromediators released by nerve cells and the cutaneous cells themselves; they can thus receive and respond to these neuromediators.

Skin aging is associated with a deregulation of the metabolism of cutaneous cells characterized by a diminishing of the proliferation of keratinocytes, a deregulation of the differentiation of keratinocytes, an accumulation of dead cells, and a diminishing of the innervation of the skin.

Alpha-MSH or alpha-melanocyte-stimulating-hormone (α-MSH), adrenocorticotropic hormone (ACTH), beta lipotropic (β-LPH), and beta endorphins are neuro-hormones generated by the single gene proopiomelanocortin (POMC). They are mainly synthesized in the pituitary gland but are also found in numerous peripheral tissues such as the skin (Wintzen M, Yaar M, Burbach JP, Gilchrest BA. J Invest Dermatol. 1996 Apr; 106(4):673-8*.).* The POMC gene and the proteins α-MSH, ACTH, β-LPH and beta endorphin are expressed by dermal cells, fibroblasts, Langerhans cells, and epidermal cells, melanocytes, and keratinocytes.

To these proteins correspond receptors which are also expressed by skin cells. Melanocortin receptor 1 (MC-1R or MCR-1) is the receptor of α-MSH and ACTH, melanocortin receptor 2 (MC-2R or MCR-2) is the receptor of ACTH, and µ opioid receptor (µ opioid R) is the receptor of β endorphin.

In the skin, α-MSH, ACTH and β endorphin inhibit the synthesis of the inflammatory cytokines IL-1, TNFα, and IL-10; they thus have anti-inflammatory properties. Moreover, these same cytokines stimulate the synthesis of these neuro-hormones (Moustafa M, Szabo M, Ghanem GE, Morandini R, Kemp EH, MacNeil S, Haycock JW. J Invest Dermatol. 2002 Dec; 119(6):1244-53*.).* Thus, there exists in the skin a negative retro-control of neurocutaneous inflammation.

α-MSH and ACTH are known to regulate melanogenesis by acting on their respective receptors in melanocytes. α-MSH and ACTH are inductors of melanocyte proliferation. In addition, β endorphin is known to have a mitogenic effect on melanocytes and increases melanocyte dendricity (Kauser S, Schallreuter KU, Thody AJ, Gummer C, Tobin DJ, J Invest Dermatol. 2003 Jun; 120(6):1073-80).

α-MSH and β endorphin are known to be synthesized by keratinocytes as well. These two proteins induce the proliferation and differentiation of skin keratinocytes *(*Chakraborty AK, Funasaka Y, Slominski A, Ermak G, Hwang J, Pawelek JM, Ichihashi M., Biochim Biophys Acta. 1996 Aug 28; 1313(2):130-8*).*

The different cells of the skin (keratinocytes, melanocytes, and fibroblasts) are capable of synthesizing other neuromediators such as neurotrophins like nerve growth factor (NGF). Keratinocytes and fibroblasts produce receptors to NGF, TrkA, and p75NTR. By attaching itself to TrkA, NGF induces the proliferation of these cells and protects keratinocytes from apoptosis. NGF is widely known for inducing neuron differentiation and permitting their survival; thus it plays an important role in the maintenance of neuronal density. Moreover, in mice the presence of the POMC mRNA has been shown in the neurons of the ganglions of the dorsal root, which suggests the involvement of the melanocortin system and notably of α-MSH in the repair of peripheral nerves. Alpha MSH would thus play a role in neurotrophicity (Gispen WH, Adan RA. Ann N Y Acad Sci. 1999 Oct 20;885:342-9*,* van der Kraan M, Tatro JB, Entwistle ML, Brakkee JH, Burbach JP, Adan RA, Gispen WH. Brain Res Mol Brain Res. 1999 Jan 8;63(2):276-86*).* Moreover, in attaching itself to the µ opioid receptors of nervous cells, beta endorphin and its derivatives are known to produce a sensation of well-being.

Up to now, professionals have sought to modulate the release of neuromediators such as α MSH or β endorphins or to mimic their role in order to affect homeostasis of the skin and/or improve innervation of the skin.

In addition, professionals are usually seeking to combat the effects of cutaneous aging through different metabolic routes that are not all comparable. However, today there remain new paths to explore.

A previous patent application WO2007/039058 has disclosed general use of opioid receptor antagonists to down-regulate the expression of the opioid receptors in melanocytes. Another previous patent application US2004/0214851 has disclosed a method for increasing expression of opioid receptors for diagnostic and/or therapeutic utility thus only in damaged tissues. Thus these disclosures do not provide with solutions to fight the effects of cutaneous aging as previously defined or recited herein after.

### Goals of the invention:

The inventors have sought innovative ways to restore innervation of the skin or maintain or promote homeostasis of skin cells as part of the general context of aging.

The main goal of the invention is to provide at least one active substance, notably at the cutaneous level, in order to:
- Promote cellular homeostasis, in particular encouraging the proliferation of keratinocytes, in order to permit re-epithelialization,
- Directly or indirectly restore or maintain innervation of the skin in order to permit maintenance of the neuritic network of the epidermis and to procure a feeling of well-being.
All of this is in order to prevent and/or fight against the effects of stress inducing variations observed during cutaneous aging, for example when aging is photo-induced or chronological.

Thus one of the goals of this invention is to prevent and/or fight against the loss of properties of the skin, particularly of the epidermis.

This invention is also intended to provide a substance permitting the cascade induction of the synthesis of other mediators involved in the innervation, and epithelialization of the skin.

This invention is also intended to provide a substance to prevent and/or fight against a loss of trophicity at the cutaneous level.

This invention is also intended to provide active substances such as described above in the domain of cosmetics.

A notable goal of this invention is to provide active substances applicable topically and/or neutraceutically being a plant extract.

### Summary of the invention:

Among the innovative paths explored by the inventors to palliate the technical problems mentioned above and attain the goals of this invention, the inventors have discovered that it was particularly interesting to increase the expression of at least one receptor of at least one mediator coded by the POMC gene at the cutaneous level.

Methods of identifying active components are based on the induction of the expression of the hormones α MSH or β endorphin or on the addition of substances that imitate the action of these neurohormones but do not in any way cause the expression of the receptors of these hormones.

The inventors have discovered that there was approximately a around 4-times diminishing of expression at the level of the genes of the receptors MC-1R, MC-2R, and µ opioid R and a around 5-times increase of the POMC gene in keratinocytes during the process of chronobiological aging.

This disclosure concerns the substances stimulating the expression of at least one of the 3 receptors MC-1R, MC-2R, and µ opioid R, particularly at the cutaneous level, in order to improve the effect of the neuromediator that will thus permit the cascade induction of the synthesis of other mediators involved in the innervation, and epithelialization of the skin.

The disclosure concerns at least one substance stimulating the expression of at least one of the genes of the 3 receptors MC-1R, MC-2R, and µ opioid R, particularly at the cutaneous level, in order to maintain or promote cellular homeostasis at the cutaneous level and to restore innervation of the skin.

By stimulation of the expression of at least one receptor MC-1R, MC-2R, or µ opioid receptor, the inventors mean the stimulation, possibly partial of at least one gene coding the protein MC-1R, MC-2R, or µ opioid R, respectively, but also the stimulation, possibly partial, of the synthesis of at least the protein MC-1R, MCR2, or the µ opioid receptor from the respective RNA messengers, as well as the stimulation, possibly partial, of the activity of the proteins MC-1R, MC-2R, or µ opioid R.

It is preferred to stimulate the expression of receptor genes around 4 times in the case of combating aging.

It is preferred to stimulate the expression of the proteins MC-1R, MC-2R, or µ opioid R around 5 times in the case of combating aging.

By the term "restore" the inventors mean, in reference to a parameter, the fact of returning from a level of this parameter inferior or superior to what would be desirable for good physiological functioning to a physiological level that is more favorable to the subject concerned.

By the term "procure a feeling of well-being" the inventors mean the well-being provoked by the liberation of beta endorphins that attach themselves easily to the µ-opioid receptor.

This disclosure particularly concerns the stimulation of the expression of the MC-1R gene and/or the MC-2R gene and/or the µ opioid R gene, and/or the proteins respectively coded by these genes, particularly in human beings.

This disclosure also particularly concerns the modulation of products of the POMC gene as well as the MC-1R and/or MC-2R receptors, and/or µ opioid R, in order to increase the effectiveness of receptor-ligand complexes; thus the active components also modulating the expression of the POMC gene are still preferable. It is possible to inhibit the expression of the POMC gene by around 5 times in the case of the fight against aging but it is preferable to maintain or increase the expression of POMC gene according to the invention to improve neuromediators effects.

The stimulation should preferably be sufficiently effective to permit the stimulation of the proliferation and/or differentiation of the targeted cutaneous cells that express at least one of these receptors, and/or restore innervation of the skin at least partially.

Active components permitting the obtaining of an expression equal to at least around 1.2 times and preferably at least around 2 times the expression of the genes MC-1R, MC-2R, and/or the µ· opioid receptor in a model comprising at least one cellular type presenting the expression of these receptors in contact with these active components in comparison with the level of expression of these receptors in a control model (one not put in contact with the active components) are considered as effectively stimulating or activating the genes of the receptors mentioned above

Active components permitting the obtaining of an expression equal to at least approximately 1.1 times, and advantageously 1.2 times the expression of the proteins MC-1R, MC-2R, and/or the µ opioid R in a model comprising at least one cellular type presenting the expression of these receptors in contact with these active components in comparison with the level of expression of these receptors in a control model (one not put in contact with the active components) are considered as effectively stimulating or activating the expression of the receptors mentioned above.

According to the method of execution, the expression of the genes MC-1R, MC-2R, and/or the µ opioid receptor, and/or the proteins MC-1R, MC-2R and/or the µ opioid receptor are activated or stimulated in the keratinocytes. Possibly it is preferable to maintain or stimulate the POMC gene and/or the neuromediators coded by the POMC gene in the keratinocytes.

According to another method of execution, the expression of at least one of these expressed receptors is increased in keratinocytes in order to diminish the inflammatory response, notably as part of a pathology related to a hyperproliferation of these cutaneous cells.

The present description will also describe a method of identification of an active substance modulating the proliferation and differentiation of at least one type of living cells capable of expressing MC-1R, MC-2R, and/or characterized µ opioid receptor characterized in that it includes:
- The putting in contact of the active substance with at least one type of living cells capable of expressing MC-1R, MC-2R, and/or the µ opioid receptor, and possibly the POMC gene;
- Analysis of the expression of MC-1R, MC-2R, and/or µ opioid receptor, with the particular objective of identifying an active substance modulating, preferably stimulating the expression of MC-1R, MC-2R and/or µ receptor.

Preferably, the analysis of the expression of the genes of the receptors MC-1R, MC-2R, and/or the µ opioid receptor, and possibly of the POMC gene, is carried out by qualitative and/or quantitative analysis of the expression of a sequence of nucleotides coding MC-1R, MC-2R, and/or the µ opioid receptor.

Preferably, the RT-PCR includes the use of primers hybridizing with at least one part of the sequence of DNA nucleotides complementary to the SEQ ID No. 1, 2, 3, 4 to amplify at least one part of the sequence of nucleotides coding MC-1R, MC-2R, the µ opioid receptor, and possibly the POMC gene.
SEQ ID N°1: MC-1R NM_002386
SEQ ID N° 2: MC-2R NM_000529
SEQ ID N° 3: µ opioid receptor NM_000914
SEQ ID N° 4: POMC NM_000939

The living cells are keratinocytes notably extracted from the skin of an adult human subject, this skin having a specific location (abdomen, breast, etc). It is preferable to use so-called "normal" cells; that is, for example, non-transformed cells (tumorous or genetically modified) and significantly representative for tests of the cellular type of the group of subjects being studied.

Preferably, categories of age are created in order to establish an age/genetic relationship and age/protein synthesis relationship. The subjects referred to as young are between 17 and 40 years of age; the subjects referred to as intermediate are between 40 and 50 years of age, and the older subjects are older than 50 years.

Preferably, the method of identification comprises a step of analysis of the expression of the proteins MC-1R (34.7 kDa), MC-2R (33.9 kDa), and/or µ opioid receptor (44.8 kDa) via the method of transfer of proteins from a gel to a nitrocellulose membrane (Western blot) notably in order to detect modulation of the expression of the corresponding proteins when the active substance is in contact with the aforementioned living cells.

According to the method of execution, the type of living cells capable of expressing MC-1R, MC-2R, and/or µ opioid receptor are keratinocytes. In this case, the active components are sought that stimulate or increase the expression of MC-1R, MC-2R, and/or µ opioid receptor, and possibly diminishing the expression of the POMC gene.

Preferably, in acting on keratinocytes, the active substance induces a cascade increase in the synthesis of NGF and VEGF, and thus stimulates innervation (density and dendricity of neurons) and neoangiogenesis, respectively.

A second aspect of the disclosure concerns the use of an active substance as described previously, as an active substance in a cosmetic composition, in order to stimulate the synthesis of the proteins MC-1R and/or MC-2R and/or µ opioid receptor, notably in order to induce the proliferation and/or differentiation and/or maturation of keratinocytes.

The invention as defined in the claims thus concerns the use of an active substance as previously described as an active substance in a cosmetic composition to prevent and/or fight against the effects of stress inducing the variations observed during the process of cutaneous aging, or prevent and/or fight against the diminishing of epidermal homeostasis as part of the general context of aging, or to improve cellular proliferation and differentiationat the epidermal level, or to generate a sensation of well-being.

The effective quantity of active substance referred to is determined by the professional through simple routine experience.

### Detailed description of the invention:

The inventors have shown for the first time that the expression of the receptors MC-1R, MC-2R, and µ opioid R diminishes during the process of chronobiological cutaneous aging when these receptors are expressed by keratinocytes in human skin.

The inventors have also identified for the first time that the expression of the POMC gene is increased during chronobiological cutaneous aging, notably in the keratinocytes in human skin.

The inventors have thus described screening process in keratinocytes to permit the search for actives among vegetable extracts, stimulating in particular the expression of mRNAs coding the receptors MC-1R, MC-2R, and µ opioid R, and possibly maintaining, stimulating or inhibiting the expression of mRNAs coding the neuromediators coded by the POMC gene. The actives selected were then tested on the expression of the proteins corresponding to the mRNAs.

The inventors have also characterized the expression of the MC-1R, MC-2R, and µ opioid receptors in keratinocytes. Only the mRNA of MC-2R has previously been identified in keratinocytes (Moustafa M, Szabo M, Ghanem GE, Morandini R, Kemp EH, MacNeil S, Haycock JW., J Invest Dermatol. 2002 Dec; 119(6):1244-53*.),* thus the inventors are the first to have identified the protein MC-2R in keratinocytes, implying the fact that ACTH carries out its activity not only by attaching itself to MC-1R in keratinocytes, but also in attaching itself to MC-2R.

Thus this disclosure describes the use of a substance stimulating the expression of at least one gene coding one receptor of a neuromediator coded by the POMC gene chosen from among MC-1R, MC-2R and µ opioid R in at least one type of skin cells expressing at least one of these receptors. This disclosure describes the use of a substance as the active substance for the preparation of a composition in order to encourage at least one interaction of one chosen neuromediator among alpha-MSH, ACTH, and beta-endorphin, with their respective receptors.

The selected actives have been incorporated into cosmeti, compositions, in particular, for application in the prevention and/or fight against the diminishing of epidermal thickness, during chronobiological or photo-induced cutaneous aging.

Preferably, the aforementioned substance increases or stimulates the expression of a gene coding a receptor of a neuromediator coded by the POMC gene to fight against or prevent a deregulation of the balance between ligand and receptor in keratinocytes.

Preferably the aforementioned substances increase or maintain the expression of the POMC gene to increase aforementioned effects of alpha-MSH, ACTH, and beta-endorphin.

The said substance increases the expression of at least one receptor of a neuromediator coded by the POMC gene in keratinocytes, to prevent and/or fight against the effects of stress inducing variations observed during cutaneous aging.

Thus this disclosure concerns the stimulation of these receptors in particular to palliate a deregulation, notably a decreasing in the interaction of alpha-MSH, and/or ACTH, and/or beta-endorphin with their respective receptors, linked to cellular aging and affecting keratinocytes.

The substance stimulating the expression of MC-1R and/or MC-2R and/or µ opioid receptor in keratinocytes is
a plant extract of Achillea millefolium (common yarrow).

According to a preferred embodiement, the plant extract is preferably obtained by macerating said plant (preferably roots, rhizomes, stems, bark, flowers, fruits, seeds, germs, or leaves) at 1-10 % (p/p), usually 1-5 % in a solvent or a mixture of solvents, usually a mixture of water and alcohol, glycol, or polyol (such as ethanol, glycerol, butylene glycol and other glycols, xylitol, etc.) 100/0 to 0/100, and preferably in water. The extracts obtained are eventually then filtered or distilled in order to recover the soluble portion, which is then filtered, preferably at 0.45µm.

The aforementioned active substances are preferably used in cosmetic compositions in an amount comprised between 0.01% and 5% (v/v) for plant extracts.

The aforementioned active substance can be used in combination with another active substance chosen from among the group consisting of:
- active components stimulating the trophicity of cutaneous nerves and/or activating sensitive cutaneous nerves, like for example those cited in the patent application FR 2825273, extract of paprika (Capsicum annuum), red pigment (Red pepper), or pepper (Piper nigrum), or glutamylamidoethylindole (Exsymol);
- the active components imitating the effect of beta endorphin in order to improve the barrier function of the skin, like for example those cited in patent application US 2006069032, extract of cocoa bean hull;
- The active components stimulating the synthesis of beta endorphin in order to give a sensation of well-being, for example Tephroline, from the plant tephrosia purpurea (Soliance);
- The active components stimulating cellular proliferation and/or cellular differentiation, intended to have anti-aging activity, such as the following molecules: NGF, α-MSH, β endorphin, or derivatives, for example those described in patent application FR 2857874, P3-endorphin.
- active components protecting Fibroblast Growth Factor against degradation, notably FGF2 such as vegetal extract described in patent application filed by Applicant published under GB244036 in particular Hibiscus Abelmoscus extract;
- active components stimulating activity and/or proliferation of fibroblasts such as a fermented peptide of soy like a product marketed by the Applicant under the tradename Phytokine ™ , optionally in combination with a Hibiscus Abelmoscus extract;
- active components stimulating Hyaluronase synthase, notably Hyaluronase synthase 2 as described in patent FR2893252,
- active components stimulating activity and/or synthesis of Lysyl Oxidase like LOXL such as components described in patent number FR2855968 in particular dill extract for stimulation of elastic fibers.
- active components with anti-inflammatory properties such as those inhibiting PLA2, and in particular components described in patent FR2847267 and notably an extract of pueraria lobata roots (Inhipase®);
- active substances permitting a change in the color of the skin, such as active components for skin lightening and/or whitening;
- active substances with draining properties, like hesperitine laurate (Flavagrum®), or quercitine caprylate(Flavenger®).

The compounds used according to the present invention are prepared in the form of topical cosmetic compositions. Thus, for these compositions, the excipient contains, for example, at least one compound chosen from among the group consisting of preservatives, emollients, emulsifiers, surfactants, moisturizers, thickeners, conditioners, mattifying agents, stabilizers, antioxidants, texturizing agents, shine agents, filmogenic agents, solubilizers, pigments, colorants, perfumes, and solar filters. These excipients are preferably chosen from among the group consisting of amino acids and their derivatives, polyglycerols, esters, polymers and cellulose derivatives, lanoline derivatives, phospholipids, lactoferrins, lactoperoxidases, sucrose-based stabilizers, vitamin E and its derivatives, natural and synthetic waxes, vegetable oils, triglycerides, insaponifiables, phytosterols, plant esters, silicones and their derivatives, protein hydrolysates, jojoba oil and its derivatives, lipo/hydrosoluble esters, betaines, aminoxides, saccharose ester plant extracts, titanium dioxides, glycines, parabens, and even more preferably from among the group consisting of butylene glycol, steareth-2, steareth-21, glycol-15 stearyl ether, cetearyl alcohol, phenoxyethanol, methylparaben, propylparaben, butylparaben, butylenes glycol, natural tocopherols, glycerine, dihydroxycetyl sodium phosphate, isopropyl hydroxycetyl ether, le glycol stearate, triisononanoin, octyl cocoate, polyacrylamide, isoparaffin, laureth-7, carbomer, propylene glycol, glycerol, bisabolol, dimethicone, sodium hydroxide, PEG 30-dipolyhydroxysterate, capric/caprylic triglycerides, cetearyl octanoate, dibutyl adipate, grapeseed oil, jojoba oil, magnesium sulfate, EDTA, cyclomethicone, xanthan gum, citric acid, sodium lauryl sulfate, mineral waxes and oils, isostearyl isostearate, dipelargonate of propylene glycol, isostearate of propylene glycol, PEG 8, beeswax, glyceride of hydrogenated palm kernel oil, lanolin oil, sesame oil, cetyl lactate, lanolin alcohol, titanium dioxide, lactose, saccharose, low-density polyethylene, and isotonic salt solution.

Ideally, the aforementioned compounds are formulated in a form chosen from among the group consisting of an aqueous or oil-based solution, a water-based creme or gel or an oily gel, usually in a jar or a tube, particularly a shower gel, shampoo, milk, emulsion, microemulsion or nanoemulsion, particularly oil-in-water or water-in-oil or multiple of silicone-based; a lotion, particularly in a glass or plastic bottle of a spray or aerosol bottle, a blister-pack, liquid soap, a dermatological bar of soap, a pomade, mousse, an anhydrous product, preferably liquid, cream or solid, for example in the form of a stick, particularly in the form of lipstick.

The term "topical application" used here means to apply or spray the composition of this invention onto the surface of the skin.

Numerous active cosmetic ingredients are known by professionals to improve the health and/or physical appearance of the skin. Professionals know how to formulate cosmetic compositions to obtain the best effects. In addition, the compounds described in this invention can have a synergistic effect when they are combined with each other. These combinations are also covered by this invention. The CTFA Cosmetic Ingredient Handbook, Second Edition (1992) describes different cosmetic and pharmaceutical ingredients currently used in the cosmetic and pharmaceutical industry that are particularly adapted to topical use. Examples of these types of ingredients include but are not limited to the following compounds: abrasives, absorbent compounds, compounds with aesthetic purposes such as perfumes, pigments, colorants, essential oils, astringents, etc. (for example: clove oil, menthol, camphor, eucalyptus oil, eugenol, menthyl lactate, and hamelis distillate), anti-acne agents, anti-flocculant agents, anti-foaming agents, anti-microbial agents (for example iodopropyl butylcarbamate), les antioxidants, bonding agents, biological additives, tampon agents, swelling agents, chelatants, additives, biocidal agents, denaturants, external analgesics, film-forming materials, polymers, opacifying agents, pH adjusters, reducing agents, depigmenting or lightening agents (for example: hydroquinone, kojic acid, ascorbic acid, magnesium ascorbyl phosphate, ascorbyl glucosamine), conditioning agents (for example: humectants), calming agents for the skin and/or scarring agents (for example: panthenol and its derivatives, for example ethyl panthenol), aloe vera, pantothenic acid and its derivatives, allantoin, bisabolol and dipotassium glycyrrhizinate), thickeners, vitamins, and the derivatives or equivalents of these.

In the figures:
Figure 1 shows a significant decrease in the expression of the genes of receptors MC-1R, MC-2R and and µ opioid receptor on human biopsies in keratinocytes during aging process.
Figure 2 shows a significant decrease in the expression of tubulin βIII and the 200 neurofilaments via a method of immunofluorescence on biopsy sections of normal human skin.
Figures 3 and 4 represent the relative rate of expression of tubulin βIII and 200 neurofilaments respectively in subjects aged between 15 and 75 years.
Figures 5 and 6 represent the statistical analysis corresponding to the studies presented in figures 3 and 4 respectively.

Other goals, characteristics, and benefits of the invention will appear clearly to professionals upon reading of the explanatory description which refers to examples given solely for the purposes of illustration and should not in any way limit the scope of the invention.

The examples are an integral part of this invention and any characteristic that appears new compared to any previous technique from the description taken in its entirety, including examples, is an integral part of the invention in its function and generality.

Thus, each example has a general scope.

### EXAMPLES

### Example 1: Demonstration of a drop in the level of expression of the proteins MC-1R. MC-2R, and µ opioid receptor on human biopsies through immunohistology during the aging process

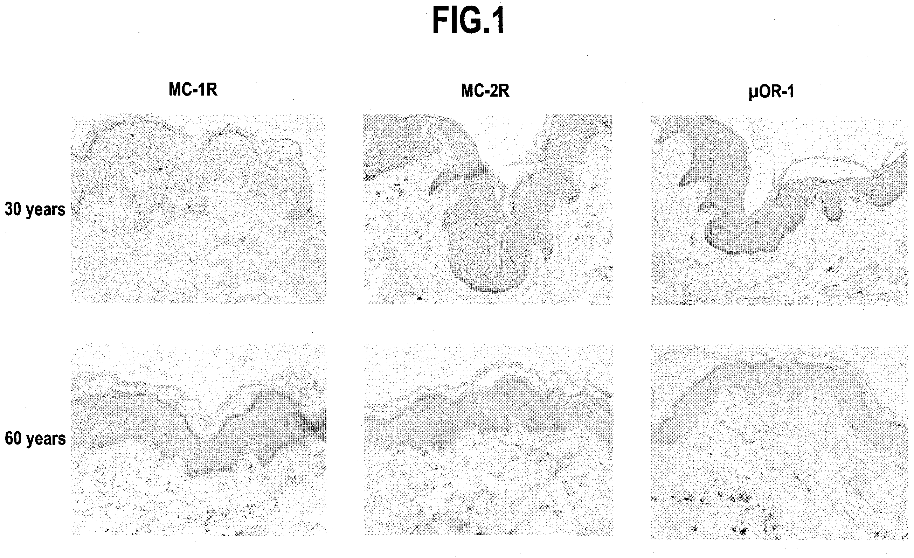

The inventors have demonstrated a variation of expression of the MC-1R, MC-2R, and µ opioid receptors at the proteic level during the process of chronobiological aging in biopsies taken from young women (younger than 40 years of age) and aged women (older than 50 years of age). (see figure 1 : Receptors expression in human biopsies form human patients aged 30 or 60 years old -immunohistochemistry results)
This study was implemented using an immunohistology study.
This demonstration was obtained without ambiguity thanks to the use of the antibodies anti-MC-1R (1/500), anti-MC-2R (1/200) and the anti- µ opioid receptor (1/1000).
The reconstructed skin model (Mimeskin®, Coletica, Lyon, France) and biopsies were prepared for immunomarkings in frozen cross-sections or after sealing in paraffin.

The antibodies used are the following: anti-MC-1R, anti-MC-2R, and anti-µ opioid receptor (Chemicon International). Detection is conducted with an appropriate conjugate secondary antibody.
Results shown in Figure 1 prove that expression at the level of the genes of the receptors MC-1R, MC-2R, and µ opioid R decreases in keratinocytes with aging.

### Example 2: Demonstration of a drop in the level of expression of the genes MC-1R. MC-2R, and µ opioid receptor and of an increase in the expression of the POMC gene in adult keratinocytes during the aging process via the RT-PCR method in real time

The invention also deals with the variation of expression of the genes of the receptors MC-1R, MC-2R, and µ opioid R and the variation of expression of the POMC gene during the process of chronobiological aging in keratinocytes taken from human biopsies. The expression of the four genes of interest as well as of the actin has been analyzed by RT-PCR in real time (reverse transcriptase polymerase quantitative chain reaction). This technique permits precise quantification of the expression of a gene by connecting it to that of the actin (considered as a constant). The regulation of the level of expression of this gene can thus be quantified.

The total RNA is purified with the "SV 96Total RNA Isolation System" kit (Promega, Charbonnières, France). The purified RNA is suspended in 100 µl of RNase-free water (Promega, Charbonnières, France), measured and divided into plates (96-well microtiter plates, 10µl of total RNA at 5ng/µl per PCR). The starters selected for use in this project are the following and the subject of table I:

**Table I:**

| | |
|---|---|
| Actin sense (SEQ ID N°5) | GTGGGGCGCCCCAGGCACCA |
| Actin antisense (SEQ ID N°6) | CTCCTTAATGTCACGCACGATTTC |
| MC-1R sense (SEQ ID N°7) | GGGCTCTGAGAACGACTTTT |
| MC-1R Antisense (SEQ ID N°8) | CCGGGCTCCTGTCTGGTTGG |
| MC-2R sense (SEQ ID N°9) | TCACGTCGCTGTTCCCGCTGAT |
| MC-2R Antisense (SEQ ID N°10) | AAGAGAGACATGTAGCAGGCGCAGTA |
| µ opioid receptor sense (SEQ ID N°11) | CTCAGCCAGGACTGGTTTCTGTAAGA |
| µ opioid receptor antisense (SEQ ID N°12) | TCGGACAGGTTGCCATCTAAGTG |
| POMC sense (SEQ ID N°13) | CGCCCAGTGAAGGTGTACCC |
| POMC Antisense (SEQ ID N°14) | GGCGTCTGGCTCTTCTCGGAGGTC |

The technique of RT-PCR in real time is conducted with the "Quanti Tect SYBR Green RT-PCR" kit (Qiagen, France) on plates containing mRNA, in an OPTICON thermocycler, which carries out cycles of amplification. Retrotranscription (RT) takes place for 30 minutes at 50°C, followed by 15 minutes at 95°C to inhibit reverse transcriptase, activate polymerase, and alter the complementary DNA (cDNA) obtained. 50 cycles of polymerization in sequence (PCR) are carried out (15 seconds at 95°C, 30 seconds at 60°C, 30 seconds at 72°C). At the end of each cycle, fluorescence, which is proportional to the number of fragments, is read. The level of expression is defined by the level of expression of each gene in comparison to actin.

The inventors have shown that the expression of the MC-1R gene is 4 times less expressed in women aged more than 52 years compared to young woman, and this in a statistically significant way.

The inventors have shown that the expression of the MC-2R gene is 8 times less expressed in women more than 5 years of age compared to young women, and this in a statistically significant way.

The inventors have shown that the expression of the gene of the µ opioid receptor is 6.5 times less expressed in women aged more than 55 years compared to young women, and this in a statistically significant way.

The inventors have shown that the expression of the POMC gene is 5 times more expressed in women aged more than 50 years compared to young women, and this in a statistically significant way.

In conclusion, the invention permits the demonstration of a disturbance of the balance between the expression of receptors and their ligand in human keratinocytes in favor of an increase of the ligand and a diminishing of all receptors.

### Example 3: Analysis of the expression of the RNA messengers of the receptors MC-1R, MC-2R and µ opioid R and of the mRNAs of POMC, for example via quantitative RT-PCR with or without the putting in contact of active components with keratinocytes

The disclosure particularly describes a method of screening new molecules capable of inducing the synthesis of the 3 receptors MC-1R, MC-2R, and/or µ opioid R with the objective of restoring a physiological expression, such as found in the keratinocytes of young skin, of the receptors noted in aged keratinocytes in reconstructed skin, biopsies of human skin.

The active component is of plant origin .

In particular, the extracts are obtained by macerating plants (preferably roots, rhizomes, stems, bark, flowers, fruits, seeds, germs, or leaves) at 1-10 % (p/p), usually 1-5 % in a solvent or a mixture of solvents, usually a mixture of water and alcohol, glycol, or polyol (such as ethanol, glycerol, butylene glycol and other glycols, xylitol, etc.) 100/0 to 0/100, and preferably in water. The extracts obtained are then filtered or distilled in order to recover the soluble portion, which is then filtered, preferably at 0.45µm.

The active component is tested for keratinocytes after dissolution in the culturing environment: usually between 0.1% and 2% (v/v) for plants. The present results are obtained with 1% in the culturing environment for the plant extract.

Keratinocytes taken from plastic surgery on normal adults through enzymatic digestion have been amplified in a specific environment for keratinocytes and then seeded, for example at 50,000 per cm² in 24-well plates and cultivated in a single layer in a definite no-serum environment. Upon convergence, the cells are put in contact with diluted actives in the culturing environment, usually for 24 hours.

In parallel, an untreated control (environment alone) and 3 positive controls (TGF-β at 1ng/ml, IL-1α at 50 pg/ml and TNF-α at 100 ng/mL) are usually carried out, for example on the same cultivation plate.

TGF-β at 1ng/ml, TNF-α at 100 ng/mL, and IL-1α at 50 pg/ml were previously tested and stimulation of mRNA synthesis of the 3 receptors induced by these 3 cytokins at these concentrations was verified through an analysis of mRNA, for example by quantatitive RT-PCR (x2 or x.08 for the 3 inducers).

After the contact time of the actives with the cells, for example 24 hours, the environments are eliminated and the cells conserved, for example by freeze-drying at-80°C after a rinsing with 7.4 pH phosphate solution. The total RNA is extracted, for example, using a 96-well extraction kit on silica columns and divided onto a 96-well spectrophotometer at 260 nm (purity indicator: dosage of proteins at 280 nm). The RNA is diluted, for example at 5ng/µl. Qualitative RT-PCR in 1 stage is carried out, for example, on 50 ng of initial RNA on a 96-well plate, on genes of actin, MC-1R, MC-2R, µ opioid R, and POMC. The specific primers of each gene are used, for example at 0.5µM, specified in table I above.

The parameters of amplification have usually been the following: The technique of RT-PCR in real time is conducted with the "Quanti Tect SYBR Green RT-PCR" kit (Qiagen, France) on wells containing mRNA, in an OPTICON thermocycler, which carries out cycles of amplification. Retrotranscription (RT) takes place for 30 minutes at 50°C, followed by 15 minutes at 95°C to inhibit reverse transcriptase, activate polymerase, and alter the complementary DNA (cDNA) obtained. 50 cycles of polymerization in sequence (PCR) are carried out (15 seconds at 95°C, 30 seconds at 60°C, and 30 seconds at 72°C). At the end of each cycle, fluorescence, which is proportional to the number of fragments amplified, is read. The level of expression is defined by comparing the expression of each gene to actin.

The level of expression of the genes MC-1R, MC-2R, µ opioid R, and POMC have been expressed in percentage of variation in comparison with those obtained for the negative control (without treatment). The active is the following and is the subject of table II.

**Table II**

| **Name (Latin)** | **Part of the plant** | **MC-1R Control X:** | **MC-2R Control X:** | **µ opioid R Control X:** | **POMC Control X:** |
|---|---|---|---|---|---|
| Achillea millefolium | Plant | 2.73 | 3.0 | 23.0 | 3.38 |

| | | | | | |
|---|---|---|---|---|---|
| "Control X" means "control multiplied by" | | | | | |

Conclusion: The active is capable of significantly increasing, under the conditions considered, the rates of mRNA in genes coding MC-1R or MC-2R or µ opioid R in keratinocytes.

### Example 4: Detection of MC-1R. MC-2R and µ opioid receptor proteins in keratinocvtes after action of active components

Electrophoresis shows the characterization of the proteins MC-1R, MC-2R, and µ opioid receptor thanks to the following respective commercial polyclonal antibodies: OPA1-15013 (Affinity bioreagents), AB5128 (Chemicon International), and AB5511 (Chemicon International).

The cells were cultivated at 37°C in an atmosphere of 5% CO₂ in the K-SFM environment K-SFM (Invitrogen) containing BPE (25 mg), EGF (2.5 µg), and normocin.

The cells were washed once with PBS solution, and the proteins were extracted at 30 min at 4°C in lysis solution (Tris 50 mM, NaCl 250 mM, pH 7.5, 1% triton), in the presence of protease inhibitors. The lysates were centrifuged for 15 minutes at 13,000 g. The floaters are diluted with Laemmli solution in the presence of beta mercaptoethanol before electrophoresis.

For immunodetection, the proteins are separated via electrophoresis in 4-12% SDS-polyacrylamide gel. The proteins were transferred onto a nitrocellulose membrane (Biorad). The membranes are then saturated for one hour at ambient temperature in TBS solution with 3% BSA. The proteins are finally immunodetected after incubation of the primary antibody at 4°C all night, followed by the secondary antibody coupled with Alexa 488 (invitrogen), 1 hour at ambient temperature.

The antibodies were used in the following dilution: 5 µg/mL anti-MC-1R, 5 µg/mL anti-MC-2R, and 1/1000 anti-µ opioid R.

Semi-quantification was carried out via image analysis. The active is the following and is the subject of table IV.

**Table IV**

| **Name** | **Part of the plant:** | **MC-2R Control X:** | **µ opioid R Control X:** |
|---|---|---|---|
| Achillea millefolium | Plant | 18.2 | 2.81 |

| | | | |
|---|---|---|---|
| "Control X" means "control multiplied by" | | | |

Conclusion: The increases induced by the active observed at gene level are confirmed by increases at the protein level.

### Example 5: Immunodetection of βIII tubulin and neurofilament 200 in human biopsies

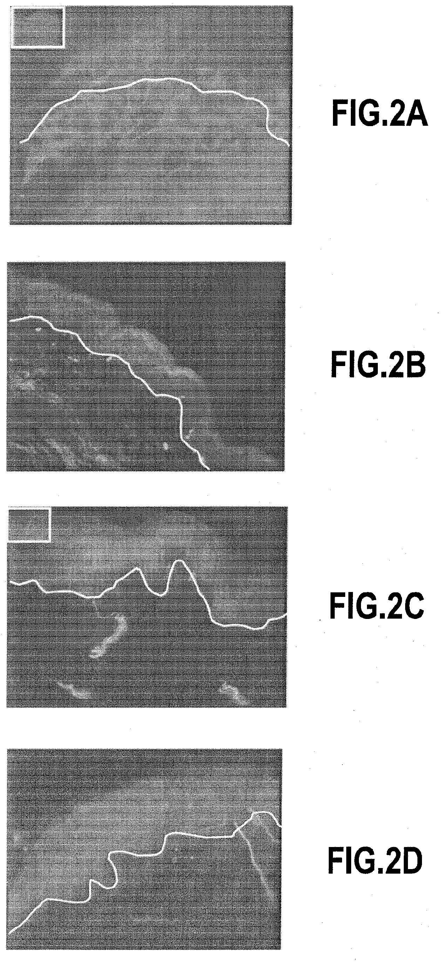

The inventors have shown that during the process of chronobiological aging, the expression of tubulin βIII and neurofilament 200 were diminished during the neurobiological aging process by immunofluorescence (fig. 2). The inventors have chosen these two markers that permit the detection of the presence of axonal prolongations in the skin. The marker tubulin βIII permits a quantification of the number of cellular cores and the density of the neuritic network, and the marker neurofilament 200 is more indicative of a maturing of the neuritic network. From an immunofluorescence study carried out on 80 mammography biopsies of women aged between 17 and 75 years, the inventors have been able to show that the expression of tubulin βIII was diminished 3.7 times after 38 years and that the expression of neurofilaments 200 was diminished by 3.5 times. The antibodies were used in the following dilution: 1:500 (anti-tubulin βIII) and 1:500 (anti-neurofilament 200). The immune complexes are detected with an anti IgG of mice (goat) joined to ALEXA 594 diluted to 1:100.

In figure 2, the detection via immunofluorescence of tubulin βIII and of neurofilaments 200 in normal human skin is represented.

Immunodetection of tubulin βIII in a so-called young subject (26 years old) is indicated in A and the immunodetection of tubulin βIII in a so-called aged subject (56 years old) is indicated in B. Immunodetection of neurofilaments 200 in a so-called young subject (26 years old) is indicated in C and the immunodetection of neurofilaments 200 in a so-called aged subject (56 years old) is indicated in D. The position of the dermo-epidermal junction is indicated by the white line. The squares on the upper left of each figure indicate a negative control using a controlled isotypical antibody.

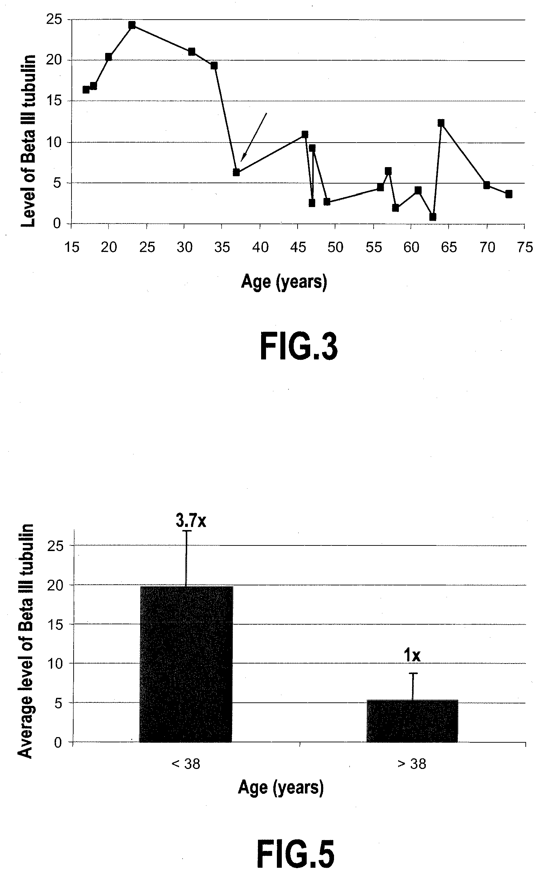

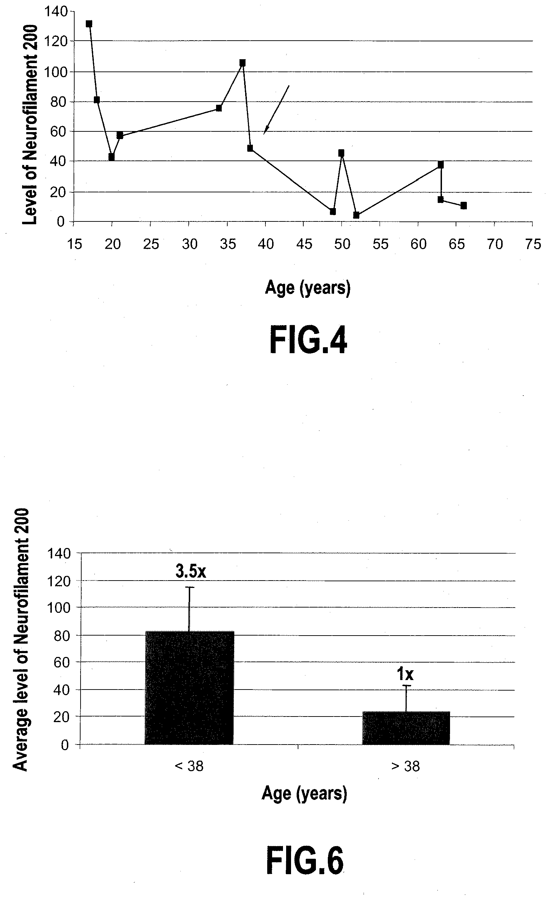

Figures 3 and 4 represent the relative rates of expression of tubulin βIII and of neurofilaments 200 respectively, in subjects aged between 15 and 75 years. The red arrow indicates the break in the curve.
Figures 5 and 6 represent the statistical analysis corresponding to the studies presented in figures 3 and 4, respectively. The results indicate that βIII tubulin and the neurofilaments 200 are 3.7 and 3.5 time (respectively) more expressed in subjects younger than 38 years of age. The results presented represent the average ± SD with a test t with P = 0.05.

### Example 6: Dosages of NGF and VEGF secreted in floaters in keratinocyte cultivation

Keratinocytes in aged subjects are put into cultivation, and after the time in contact of the actives with the cells, for example 24 hours, the environments are recovered and subjected to an ELISA technique permitting the detection of the secretion of NGF or VEGF. The method of use is in accordance with the protocol undergone by the R and D system provider (DY256) for NGF and Clinisciences (KHG0112) for VEGF. Among the actives tested, the following active present the strongest modulation, in particular of NGF, and is the subject of table V.

**Table V**

| **Plant** | **Part of the plant** | **NGF control multiplied by** |
|---|---|---|
| Achillea millefolium | Plant | 11.28 |

### Example 7: Immunodetection of beta III tubulin and neurofilament 200 in surviving human biopsies

Biopsies of adult plastic surgery are maintained in survival for 4 days in an environment of DMEM cultivation, in the presence or not of NGF or of an active component. The biopsies are then frozen and subject to immunofluorescence studies for the identification of neuronal markers, in particular those indicated in example 5.

### Example 8: Immunodetection of differentiation and proliferation markers in models of reconstructed skin.

This model is the association of a cultivation of a reconstructed dermis on which the supplementary cultivation of a reconstructed epidermis is then carried out.
The reconstructed dermis model is created according to the following protocol:
- 0.5 to 1.10⁶ normal human skin fibroblasts are seeded on a collagen-based substratum matrix, usually of glycosaminoglycane chitosan, and then cultivated in a nutritive environment, for example DMEM-Glutamax supplemented with 10% calf serum, ascorbic acid, and preferably at a final concentration of 1mM EGF (epidermal growth factor), and preferably at a final concentration of 10 ng/mL Normocin, and preferably at a final concentration of 100 µg/mL, for 21 days.

The reconstructed skin model is created according to the following protocol:
- 0.5 to 1.10⁶ normal human keratinocytes are seeded on the dermal equivalent, then cultivated in a nutritive environment, for example DMEM-Glutamax/Ham F-12 (ratio 3/1 v/v) supplemented with calf serum, ascorbic acid, and preferably at a final concentration of 1 mM, EGF (*epidermal growth factor*) and preferably at a final concentration of 10 ng/mL hydrocortisone and preferably at a final concentration of 0.4 µg/mL umuline and preferably at a final concentration of 0.12 UI/mL isuprel and preferably at a final concentration of 0.4 µg/mL triiodothyronine and preferably at a final concentration of 2.10⁻⁹ M adenine and preferably at a final concentration of 24.3 µg/mL Normocin and preferably at a final concentration of 100 µg/mL. Cultivation takes place for 7 days in immersed conditions. The cultures are then placed in an air-liquid interface for 14 additional days in the same environment as the culture in immersion, except for the calf serum, hydrocortisone, isuprel, triiodothyronine, and umuline.

The reconstructed skin (Mimeskin®, Coletica, Lyon, France) was then prepared in Boulin fixing solution (LOX, LOXL, elastin) or in a 10% formol solution (for elastin), then sealed in paraffin for an immunihistochemical study, or directly frozen in liquid nitrogen for analysis of immunofluorescence. 6 µm-thick sections were extracted from the paraffin and bleached in glycine-HCl (100 mmol/l). Immunodetections of Ki67 (proliferation marker), Keratin 14 (marker of all cellular layers), keratin 10 (marker of suprabasal keratinocyte layers), involucrine, tranglutaminase, and nestine were carried out on the reconstructed skin on day 45.

### Example 9: Use of product of the invention in combination with existing patented molecules

It is possible to combine the product of the invention with, in particular, extracts stimulating the trophicity of cutaneous nerves active on sensitive cutaneous nerves, like for example paprika extract (Capsicum annuum) and/or red pigment (red pepper) and/or pepper (Piper nigrum) (L'OREAL FR2825273), and or glutamylamidoethylindole (Exsymol).

It is feasible to combine the product of the invention with extracts that imitate the action of β endorphin in order to improve the barrier function of the skin, like for example extract of hull of cocoa bean (L'OREAL US2006069032).

It is also feasible to combine the product of the invention with extracts that activate β endorphin in order to generate a feeling of well-being, like for example extract of Tephrosia purpurea (faux indigo).

It is also feasible to combine the product of the invention with other molecules such as α-MSH, β endorphin, or derivatives such as, for example, P3-endorphin and essential oils in order to stimulate the differentiation of keratinocytes intended to have an anti-aging effect (CODIF FR2857874) of NGF in order to have an effect on nerve trophicity.

### Example 10: Use of products of the invention in cosmetic or pharmaceutical formulations of the oil-in-water emulsion type

### Formulation 10a:

| | | |
|---|---|---|
| A | Water | qsp 100 |
| | Butylene Glycol | 2 |
| | Glycerine | 3 |
| | Sodium Dihydroxycetyl Phosphate, Isopropyl Hydroxycetyl Ether | 2 |
| | | |
| B | Glycol Stearate SE | 14 |
| | Triisononaoin | 5 |
| | Octyl Cocoate | 6 |
| | | |
| C | Butylene Glycol, Methylparaben, Ethylparaben, Propylparaben, pH adjusted to 5.5 | 2 |
| | | |
| D | Products of the invention | 0.01 - 10 % |

### Formulation 10b:

| | | |
|---|---|---|
| A | Water | qsp 100 |
| | Butylene Glycol | 2 |
| | Glycerine | 3 |
| | Polyacrylamide, Isoparafin, Laureth-7 | 2.8 |
| | | |
| B | Butylene Glycol, Methylparaben, Ethylparaben, Propylparaben; | 2 |
| | | |
| | Phenoxyethanol, Methylparaben, Propylparaben, Butylparaben, | 2 |
| | Ethylparaben | 0.5 |
| | | |
| | Butylene Glycol | |
| | | |
| D | Products of the invention | 0.01 - 10 % |

### Formulation 10c:

| | | |
|---|---|---|
| A | Carbomer | 0.50 |
| | Propylene Glycol | 3 |
| | Glycerol | 5 |
| | Water | qsp 100 |
| | | |
| B | Octyl Cocoate | 5 |
| | Bisabolol | 0.30 |
| | Dimethicone | 0.30 |
| | | |
| C | Sodium Hydroxide | 1.60 |
| | | |
| D | Phenoxyethanol, Methylparaben, Propylparaben, Butylparaben, Ethylparaben | 0.50 |
| E | Perfume | 0.30 |
| | | |
| F | Products of the invention | 0.01 - 10 % |

### Example 11: Use of products of the invention in a formulation of the water-in-oil type

| | | |
|---|---|---|
| A | PEG 30 - dipolyhydroxystearate | 3 |
| | Capric Triglycerides | 3 |
| | Cetearyl Octanoate | 4 |
| | Dibutyl Adipate | 3 |
| | Grape Seed Oil | 1.5 |
| | Jojoba Oil | 1.5 |
| | Phenoxyethanol, Methylparaben, Propylparaben, Butylparaben, Ethylparaben | 0.5 |
| | | |
| B | Glycerine | 3 |
| | Butylene Glycol | 3 |
| | Magnesium Sulfate | 0.5 |
| | EDTA | 0.05 |
| | Water | qsp 100 |
| | | |
| C | Cyclomethicone | 1 |
| | Dimethicone | 1 |
| | | |
| D | Perfume | 0.3 |
| | | |
| E | Products of the invention | 0.01 - 10 % |

### Example 12: Use of products of the invention in a formulation of the shampoo or shower gel type

| | | |
|---|---|---|
| A | Xanthan Gum | 0.8 |
| | Water | qsp 100 |
| | | |
| B | Butylene Glycol, Methylparaben, Ethylparaben, Propylparaben | 0.5 |
| | Phenoxyethanol, Methylparaben, Propylparaben, Butylparaben, Ethylparaben | 0.5 |
| | | |
| C | Citric acid | 0.8 |
| | | |
| D | Sodium Laureth Sulfate | 40.0 |
| | | |
| E | Products of the invention | 0.01 - 10 % |

### Example 13: Use of products of the invention in a formulation of the lipstick type or other anhydrous products

| | | |
|---|---|---|
| A | Mineral Wax | 17.0 |
| | Isostearyl Isostearate | 31.5 |
| | Propylene Glycol Dipelargonate | 2.6 |
| | Propylene Glycol Isostearate | 1.7 |
| | PEG 8 Beeswax | 3.0 |
| | Hydrogenated Palm Kernel Oil Glycerides, | 3.4 |
| | Hydrogenated Palm Glycerides | |
| | Lanoline Oil | 3.4 |
| | Sesame Oil | 1.7 |
| | Cetyl Lactate | 1.7 |
| | Mineral Oil, Lanolin Alcohol | 3.0 |
| | | |
| B | Castor Oil | qsp 100 |
| | Titanium Dioxide | 3.9 |
| | CI 15850:1 | 0.616 |
| | CI 45410:1 | 0.256 |
| | CI 19140:1 | 0.048 |
| | CI 77491 | 2.048 |
| | | |
| C | Products of the invention | 0.01 - 5 % |

### Example 14: Use of products of the invention in a formulation of aqueous gels (eye contouring, slimming, etc.)

| | | |
|---|---|---|
| A | Water | qsp 100 |
| | Carbomer | 0.5 |
| | Butylene Glycol | 15 |
| | Phenoxyethanol, Methylparaben, Propylparaben, Butylparaben, Ethylparaben | 0.5 |
| | | |
| B | Products of the invention | 0.01 - 10 % |

### Example 15: Use of products of the invention in a formulation of the triple-emulsion type

**Primary emulsion W1/O**

| | | |
|---|---|---|
| A | PEG 30 - | 4 |
| | dipolyhydroxystearate | |
| | Capric Triglycerides | 7.5 |
| | Isohexadecane | 15 |
| | PPG-15 Stearyl ether | 7.5 |
| | | |
| B | Water | 65.3 |
| | | |
| C | Phenoxyethanol, Methylparaben, Propylparaben, Butylparaben, Ethylparaben | 0.7 |

**Secondary emulsion W1/O/W2**

| | | |
|---|---|---|
| A | Primary emulsion | 60 |
| | | |
| B | Poloxamer 407 | 2 |
| | Phenoxyethanol, Methylparaben, Propylparaben,2-bromo-2nitropropane-1,3 diol | 0.3 |
| | Water | qsp 100 |
| | | |
| C | Carbomer | 15 |
| | | |
| D | Triethanolamine | PH 6.0-6.5 |

### Example 16: Preparation of pharmaceutical formulations containing the product of the invention

### Formulation 176a: preparation of pills

| | | |
|---|---|---|
| A | Excipients | In g per pill |
| | Lactose | 0.359 |
| | Saccharose | 0.240 |
| B | Products of the invention* | 0.001 -0.1 |

| | | |
|---|---|---|
| *The product of the invention is obtained, for example, according to the extraction procedure described in example 3 followed by a drying stage. | | |

### Formulation 176b: preparation of a pomade

| A | Excipients | |
|---|---|---|
| | Low-density polyethylene | 5.5 |
| | Liquid paraffin | qsp 100 |
| | | |
| B | Products of the invention* | 0.001 -0.1 |

| | | |
|---|---|---|
| *The product of the invention is obtained, for example, according to the extraction procedure described in example 3 followed by a drying stage. | | |

### Formulation 16 c: preparation of an injectable formula

| A | Excipient | |
|---|---|---|
| | Salt isotonic solution | 5 ml |
| | | |
| B | Products of the invention* | 0.001 -0.1 g |

| | | |
|---|---|---|
| *The product of the invention is obtained, for example, according to the extraction procedure described in example 3 followed by a drying stage. | | |

| | | | | |
|---|---|---|---|---|
| Salsaparilla (Smilax ornata) | root | 3.9 | 1.0 | 2.74 |
| Juniperus communis | fruit | 0.4 | 1.0 | 0.25 |
| Cecropia obtusia | buds | 3.2 | 1.0 | 2.18 |
| Papaya lactic bacteria | | 2.58 | 1.0 | 1.0 |
| Carrot | fruit | 1.97 | 1.0 | 1.0 |
| Sour cherry | fruit | 2.0 | 1.0 | 1.0 |
| Lupin lactobacillus plantarum | | 1.0 | 1.0 | 2.15 |
| Soy flour | // | 1.0 | 1.0 | 2.42 |
| Date lactobacillus plantarum | fruit | 3.23 | 1.0 | 1.0 |
| Soy lactobacillus plantarum | | 2.0 | 1.0 | 1.0 |

| | | | | |
|---|---|---|---|---|
| "Control X" means "control multiplied by" | | | | |

Conclusion: 10 actives increase the expression of the MC-1R gene in melanocytes; 9 actives increase the expression of the POMC gene, and 2 actives diminish the expression of the POMC gene in melanocytes.

### Example 5: Detection of MC-1R, MC-2R and µ opioid receptor proteins in keratinocytes after action of active components

Electrophoresis shows the characterization of the proteins MC-1R, MC-2R, and µ opioid receptor thanks to the following respective commercial polyclonal antibodies: OPA1-15013 (Affinity bioreagents), AB5128 (Chemicon International), and AB5511 (Chemicon International).

The cells were cultivated at 37°C in an atmosphere of 5% CO₂ in the K-SFM environment K-SFM (Invitrogen) containing BPE (25 mg), EGF (2.5 µg), and normocin.

The cells were washed once with PBS solution, and the proteins were extracted at 30 min at 4°C in lysis solution (Tris 50 mM, NaCl 250 mM, pH 7.5, 1% triton), in the presence of protease inhibitors. The lysates were centrifuged for 15 minutes at 13,000 g. The floaters are diluted with Laemmli solution in the presence of beta mercaptoethanol before electrophoresis.

For immunodetection, the proteins are separated via electrophoresis in 4-12% SDS-polyacrylamide gel. The proteins were transferred onto a nitrocellulose membrane (Biorad). The membranes are then saturated for one hour at ambient temperature in TBS solution with 3% BSA. The proteins are finally immunodetected after incubation of the primary antibody at 4°C all night, followed by the secondary antibody coupled with Alexa 488 (invitrogen), 1 hour at ambient temperature.

The antibodies were used in the following dilution: 5 µg/mL anti-MC-1R, 5 µg/mL anti-MC-2R, and 1/1000 anti-µ opioid R.

Semi-quantification was carried out via image analysis. The actives are the following and are the subject of table IV.

**Table IV**

| **Name** | **Part of the plant:** | **MC-2R Control X:** | **µ opioid R Control X:** |
|---|---|---|---|
| Achillea millefolium | Plant | 18.2 | 2.81 |
| Galeopsis ochroleuca | Plant | 1.0 | 2.54 |
| Colocasia esculenta | tuber | 1.0 | 1.52 |
| Prunus cerasus | Stem | 1.0 | 1.44 |
| Oenothera biennis | seed | 1.0 | 1.46 |
| Prunus spinosa | fruit | 8.26 | 2.02 |
| (Z)-decahydro-6 ,8a-dihydroxy-1 -isopropyl-3a, 6-dimethylazulen-5-yl 2-methylbut-2-enoate | | 1.99 | 1.37 |
| Phenyl-acetic acid 3,6,9-trimethyl-2,7 -dioxo-2,3,3a,4,5,7,9a,9b-octahydroazuleno[4,5-b]furan-4-yl | | 1.96 | 1.66 |

| | | | |
|---|---|---|---|
| "Control X" means "control multiplied by" | | | |

Conclusion: The increases induced by the actives observed at gene level are confirmed by increases at the protein level. Galeopsis ochroleuca, Oenothera biennis and the two characterized molecules induce an increase in the expression of the protein µ opioid R though the expression of the gene is not increased.

### Example 6: Immunodetection of βIII tubulin and neurofilament 200 in human biopsies

The inventors have shown that during the process of chronobiological aging, the expression of tubulin βIII and neurofilament 200 were diminished during the neurobiological aging process by immunofluorescence (fig. 2). The inventors have chosen these two markers that permit the detection of the presence of axonal prolongations in the skin. The marker tubulin βIII permits a quantification of the number of cellular cores and the density of the neuritic network, and the marker neurofilament 200 is more indicative of a maturing of the neuritic network. From an immunofluorescence study carried out on 80 mammography biopsies of women aged between 17 and 75 years, the inventors have been able to show that the expression of tubulin βIII was diminished 3.7 times after 38 years and that the expression of neurofilaments 200 was diminished by 3.5 times. The antibodies were used in the following dilution: 1:500 (anti-tubulin βIII) and 1:500 (anti-neurofilament 200). The immune complexes are detected with an anti IgG of mice (goat) joined to ALEXA 594 diluted to 1:100.

In figure 2, the detection via immunofluorescence of tubulin βIII and of neurofilaments 200 in normal human skin is represented.

Immunodetection of tubulin βIII in a so-called young subject (26 years old) is indicated in A and the immunodetection of tubulin βIII in a so-called aged subject (56 years old) is indicated in B. Immunodetection of neurofilaments 200 in a so-called young subject (26 years old) is indicated in C and the immunodetection of neurofilaments 200 in a so-called aged subject (56 years old) is indicated in D. The position of the dermo-epidermal junction is indicated by the white line. The squares on the upper left of each figure indicate a negative control using a controlled isotypical antibody.

Figures 3 and 4 represent the relative rates of expression of tubulin βIII and of neurofilaments 200 respectively, in subjects aged between 15 and 75 years. The red arrow indicates the break in the curve.
Figures 5 and 6 represent the statistical analysis corresponding to the studies presented in figures 3 and 4, respectively. The results indicate that βIII tubulin and the neurofilaments 200 are 3.7 and 3.5 time (respectively) more expressed in subjects younger than 38 years of age. The results presented represent the average ± SD with a test t with P = 0.05.

### Example 7: Dosages of NGF and VEGF secreted in floaters in keratinocvte cultivation

Keratinocytes in aged subjects are put into cultivation, and after the time in contact of the actives with the cells, for example 24 hours, the environments are recovered and subjected to an ELISA technique permitting the detection of the secretion of NGF or VEGF. The method of use is in accordance with the protocol undergone by the R and D system provider (DY256) for NGF and Clinisciences (KHG0112) for VEGF. Among the actives tested, the following actives present the strongest modulation, in particular of NGF, and are the subject of table V.

**Table V**

| **Plant** | **Part of the plant** | **NGF control multiplied by** |
|---|---|---|
| Achillea millefolium | Plant | 11.28 |
| Prunus cerasus | Stem | 17.8 |
| Galeopsis ochroleuca | Plant | 11.24 |

### Example 8: Immunodetection of beta III tubulin and neurofilament 200 in surviving human biopsies

Biopsies of adult plastic surgery are maintained in survival for 4 days in an environment of DMEM cultivation, in the presence or not of NGF or of an active component. The biopsies are then frozen and subject to immunofluorescence studies for the identification of neuronal markers, in particular those indicated in example 6.

### Example 9: Immunodetection of differentiation and proliferation markers in models of reconstructed skin.

This model is the association of a cultivation of a reconstructed dermis on which the supplementary cultivation of a reconstructed epidermis is then carried out.

The reconstructed dermis model is created according to the following protocol:
- 0.5 to 1.10⁶ normal human skin fibroblasts are seeded on a collagen-based substratum matrix, usually of glycosaminoglycane chitosan, and then cultivated in a nutritive environment, for example DMEM-Glutamax supplemented with 10% calf serum, ascorbic acid, and preferably at a final concentration of 1mM EGF (epidermal growth factor), and preferably at a final concentration of 10 ng/mL Normocin, and preferably at a final concentration of 100 µg/mL, for 21 days.

The reconstructed skin model is created according to the following protocol:
- 0.5 to 1.10⁶ normal human keratinocytes are seeded on the dermal equivalent, then cultivated in a nutritive environment, for example DMEM-Glutamax/Ham F-12 (ratio 3/1 v/v) supplemented with calf serum, ascorbic acid, and preferably at a final concentration of 1 mM, EGF (*epidermal growth factor*) and preferably at a final concentration of 10 ng/mL hydrocortisone and preferably at a final concentration of 0.4 µg/mL umuline and preferably at a final concentration of 0.12 UI/mL isuprel and preferably at a final concentration of 0.4 µg/mL triiodothyronine and preferably at a final concentration of 2.10⁻⁹ M adenine and preferably at a final concentration of 24.3 µg/mL Normocin and preferably at a final concentration of 100 µg/mL. Cultivation takes place for 7 days in immersed conditions. The cultures are then placed in an air-iiquid interface for 14 additional days in the same environment as the culture in immersion, except for the calf serum, hydrocortisone, isuprel, triiodothyronine, and umuline.

The reconstructed skin (Mimeskin®, Coletica, Lyon, France) was then prepared in Boulin fixing solution (LOX, LOXL, elastin) or in a 10% formol solution (for elastin), then sealed in paraffin for an immunihistochemical study, or directly frozen in liquid nitrogen for analysis of immunofluorescence. 6 µm-thick sections were extracted from the paraffin and bleached in glycine-HCI (100 mmol/l). Immunodetections of Ki67 (proliferation marker), Keratin 14 (marker of all cellular layers), keratin 10 (marker of suprabasal keratinocyte layers), involucrine, tranglutaminase, and nestine were carried out on the reconstructed skin on day 45.

### Example 10: Use of the products in combination with existing patented molecules

It is possible to combine products of the invention with, in particular, extracts stimulating the trophicity of cutaneous nerves active on sensitive cutaneous nerves, like for example paprika extract (Capsicum annuum) and/or red pigment (red pepper) and/or pepper (Piper nigrum) (L'OREAL FR2825273), and or glutamylamidoethylindole (Exsymol).

It is feasible to combine products of the invention with extracts that imitate the action of β endorphin in order to improve the barrier function of the skin, like for example extract of hull of cocoa bean (L'OREAL US2006069032).

It is also feasible to combine products of the invention with extracts that activate β endorphin in order to generate a feeling of well-being, like for example extract of Tephrosia purpurea (faux indigo).

It is also feasible to combine products of the invention with other molecules such as α-MSH, β endorphin, or derivatives such as, for example, P3-endorphin and essential oils in order to stimulate the differentiation of keratinocytes intended to have an anti-aging effect (CODIF FR2857874) of NGF in order to have an effect on nerve trophicity.

### Example 11: Use of the products in cosmetic or pharmaceutical formulations of the oil-in-water emulsion type

### Formulation 11a:

| | | |
|---|---|---|
| A | Water | qsp 100 |
| | Butylene Glycol | 2 |
| | Glycerine | 3 |
| | Sodium Dihydroxycetyl Phosphate, Isopropyl Hydroxycetyl Ether | 2 |
| | | |
| B | Glycol Stearate SE | 14 |
| | Triisononaoin | 5 |
| | Octyl Cocoate | 6 |
| | | |
| C | Butylene Glycol, Methylparaben, Ethylparaben, Propylparaben, pH adjusted to 5.5 | 2 |
| | | |
| D | Products of the invention | 0.01 - 10 % |

### Formulation 11b:

| | | |
|---|---|---|
| A | Water | qsp 100 |
| | Butylene Glycol | 2 |
| | Glycerine | 3 |
| | Polyacrylamide, Isoparafin, Laureth-7 | 2.8 |
| | | |
| B | Butylene Glycol, Methylparaben, Ethylparaben, Propylparaben; | 2 |
| | Phenoxyethanol, Methylparaben, Propylparaben, Butylparaben, | 2 |
| | Ethylparaben Butylene Glycol | 0.5 |
| D | Products of the invention | 0.01 - 10 % |

### Formulation 11c:

| | | |
|---|---|---|
| A | Carbomer | 0.50 |
| | Propylene Glycol | 3 |
| | Glycerol | 5 |
| | Water | qsp 100 |
| | | |
| B | Octyl Cocoate | 5 |
| | Bisabolol | 0.30 |
| | Dimethicone | 0.30 |
| | | |
| C | Sodium Hydroxide | 1.60 |
| | | |
| D | Phenoxyethanol, Methylparaben, Propylparaben, Butylparaben, Ethylparaben | 0.50 |
| E | Perfume | 0.30 |
| | | |
| F | Products of the invention | 0.01 - 10 % |

### Example 12: Use of the products in a formulation of the water-in-oil type

| | | |
|---|---|---|
| A | PEG 30 - dipolyhydroxystearate | 3 |
| | Capric Triglycerides | 3 |
| | Cetearyl Octanoate | 4 |
| | Dibutyl Adipate | 3 |
| | Grape Seed Oil | 1.5 |
| | Jojoba Oil | 1.5 |
| | Phenoxyethanol, Methylparaben, Propylparaben, Butylparaben, Ethylparaben | 0.5 |
| | | |
| B | Glycerine | 3 |
| | Butylene Glycol | 3 |
| | Magnesium Sulfate | 0.5 |
| | EDTA | 0.05 |
| | Water | qsp 100 |
| | | |
| C | Cyclomethicone | 1 |
| | Dimethicone | 1 |
| | | |
| D | Perfume | 0.3 |
| | | |
| E | Products of the invention | 0.01 - 10 % |

### Example 13: Use of the products in a formulation of the shampoo or shower gel type

| | | |
|---|---|---|
| A | Xanthan Gum | 0.8 |
| | Water | qsp 100 |
| | | |
| B | Butylene Glycol, Methylparaben, Ethylparaben, Propylparaben | 0.5 |
| | Phenoxyethanol, Methylparaben, Propylparaben, Butylparaben, Ethylparaben | 0.5 |
| | | |
| C | Citric acid | 0.8 |
| | | |
| D | Sodium Laureth Sulfate | 40.0 |
| | | |
| E | Products of the invention | 0.01 - 10 % |

### Example 14: Use of the products in a formulation of the lipstick type or other anhydrous products

| | | |
|---|---|---|
| A | Mineral Wax | 17.0 |
| | Isostearyl Isostearate | 31.5 |
| | Propylene Glycol Dipelargonate | 2.6 |
| | Propylene Glycol Isostearate | 1.7 |
| | PEG 8 Beeswax | 3.0 |
| | Hydrogenated Palm Kernel Oil Glycerides, | 3.4 |
| | Hydrogenated Palm Glycerides Lanoline Oil | 3.4 |
| | Sesame Oil | 1.7 |
| | Cetyl Lactate | 1.7 |
| | Mineral Oil, Lanolin Alcohol | 3.0 |
| | | |
| B | Castor Oil | qsp 100 |
| | Titanium Dioxide | 3.9 |
| | CI 15850:1 | 0.616 |
| | CI 45410:1 | 0.256 |
| | CI 19140:1 | 0.048 |
| | CI 77491 | 2.048 |
| | | |
| C | Products of the invention | 0.01 - 5 % |

### Example 15: Use of the products in a formulation of aqueous gels (eye contouring, slimming, etc.1

| | | |
|---|---|---|
| A | Water | qsp 100 |
| | Carbomer | 0.5 |
| | Butylene Glycol | 15 |
| | Phenoxyethanol, Methylparaben, Propylparaben, Butylparaben, Ethylparaben | 0.5 |
| | | |
| B | Products of the invention | 0.01 - 10 % |

### Example 16: Use of the products in a formulation of the triple-emulsion type

**Primary emulsion W1/O**

| | | |
|---|---|---|
| A | PEG 30 - dipolyhydroxystearate | 4 |
| | Capric Triglycerides | 7.5 |
| | Isohexadecane | 15 |
| | PPG-15 Stearyl ether | 7.5 |
| | | |
| B | Water | 65.3 |
| C | Phenoxyethanol, Methyl para ben, Propylparaben, Butylparaben, Ethylparaben | 0.7 |

**Secondary emulsion W1/O/W2**

| | | |
|---|---|---|
| A | Primary emulsion | 60 |
| | | |
| B | Poloxamer 407 | 2 |
| | Phenoxyethanol, Methylparaben, Propylparaben,2-bromo-2nitropropane-1,3 diol | 0.3 |
| | Water | qsp 100 |
| | | |
| C | Carbomer | 15 |
| | | |
| D | Triethanolamine | PH 6.0-6.5 |

### Illustrative Example 17: Preparation of pharmaceutical formulations containing the product

### Formulation 17 a: preparation of pills

***The product of the invention is obtained, for example, according to the extraction procedure described in example 3 followed by a drying stage.**

| A | Excipients | In g per pill |
|---|---|---|
| | Lactose | 0.359 |
| | Saccharose | 0.240 |
| | | |
| B | Products of the invention* | 0.001 -0.1 |

### Formulation 17 b: preparation of a pomade

| A | Excipients | |
|---|---|---|
| | Low-density polyethylene | 5.5 |
| | Liquid paraffin | qsp 100 |
| | | |
| B | Products of the invention* | 0.001 -0.1 |

| | | |
|---|---|---|
| *The product of the invention is obtained, for example, according to the extraction procedure described in example 3 followed by a drying stage. | | |

### Formulation 17 c: preparation of an injectable formula

| | | |
|---|---|---|
| A | Excipient Salt isotonic solution | 5 ml |
| | | |
| B | Products of the invention* | 0.001 -0.1 g |

| | | |
|---|---|---|
| *The product of the invention is obtained, for example, according to the extraction procedure described in example 3 followed by a drying stage. | | |

## Claims

1. Use of at least one substance stimulating the expression of a receptor of a neuromediator coded by the POMC gene chosen from among MC-1R, MC-2R, and µ opioid R, in at least one type of skin cells expressing at least one of these receptors and a product of the POMC gene, as an active ingredient in a cosmetic composition, wherein the said substance is an extract of common yarrow plant (*Achillea millefolium*), to prevent or fight against the effects of stress inducing the variations observed during the process of cutaneous aging, and/or to prevent and/or fight against the diminishing of epidermal homeostasis as part of the general context of aging, and/ or to improve cellular proliferation and differentiation, at the epidermal level, and/or to prevent and/or fight against the diminishing of epidermal thickness during chronobiological or photo-induced cutaneous aging or to generate a sensation of well-being, and wherein the skin cells are keratinocytes.

2. Use according to claim 1, wherein the said substance also increases or maintains the POMC expression.

3. Use according to any of claim 1 or 2, to stimulate the expression of said receptor to increase cosmetical effects of alpha-MSH and/or ACTH and/or β - endorphin.

4. Use according to one of the previous claims to fight against or prevent a deregulation of the effects of alpha-MSH and/or ACTH and/or β -endorphin.

5. Use according to anyone of the previous claims, **characterized in that** said substance is a plant extract, and is used in cosmetic composition in an amount comprised between 0.01% and 5% (v/v).

6. Use according to anyone of the previous claims, **characterized in that** said substance is a plant extract obtained by macerating said plant at 1-10% (p/p) in a solvent or a mixture of solvents.

7. Use according to claim 6, **characterized in that** the mixture of solvents is a mixture of water and alcohol, glycol or polyol 100/0 to 0/100.

8. Use according to claim 6, **characterized in that** said solvent is water.

## Patentansprüche

1. Verwendung mindestens einer Substanz, welche die Expression eines Rezeptors eines Neuromediators stimuliert, der von dem POMC-Gen kodiert wird, ausgewählt aus MC-1R, MC-2R und µ-Opioid-R, in mindestens einer Art von Hautzellen, die mindestens einen dieser Rezeptoren und ein Produkt des POMC-Gens exprimieren, als Wirkstoff in einer kosmetischen Zusammensetzung, wobei die Substanz ein Extrakt der gemeinen Schafgarbe (*Achillea millefolium*) ist, um die Auswirkungen von Stress zu verhindern oder zu bekämpfen, der die Schwankungen hervorruft, die während des Prozesses der Hautalterung beobachtet werden, und/oder um die Verringerung der epidermalen Homöostase als Teil des allgemeinen Kontexts der Alterung zu verhindern und/oder zu bekämpfen, und/oder um die Zellproliferation und -differenzierung auf der Ebene der Epidermis zu verbessern, und/oder um die Verringerung der Dicke der Epidermis während der chronobiologischen oder photoinduzierten Hautalterung zu verhindern und/oder zu bekämpfen oder um ein Gefühl des Wohlbefindens zu erzeugen, und wobei die Hautzellen Keratinozyten sind.

2. Verwendung nach Anspruch 1, wobei die Substanz auch die POMC-Expression erhöht oder beibehält.

3. Verwendung nach einem der Ansprüche 1 oder 2, um die Expression des Rezeptors zu stimulieren, um kosmetische Effekte von alpha-MSH und/oder ACTH und/oder β-Endorphin zu erhöhen.

4. Verwendung nach einem der vorhergehenden Ansprüche, um eine Deregulierung der Effekte von alpha-MSH und/oder ACTH und/oder β-Endorphin zu bekämpfen oder zu verhindern.

5. Verwendung nach einem der vorhergehenden Ansprüche, **dadurch gekennzeichnet, dass** die Substanz ein Pflanzenextrakt ist und in einer kosmetischen Zusammensetzung in einer Menge zwischen 0,01 % und 5 % (v/v) verwendet wird.

6. Verwendung nach einem der vorhergehenden Ansprüche, **dadurch gekennzeichnet, dass** die Substanz ein Pflanzenextrakt ist, der durch das Aufschließen der Pflanze bei 1-10 % (p/p) in einem Lösungsmittel oder einem Gemisch von Lösungsmitteln erhalten wird.

7. Verwendung nach Anspruch 6, **dadurch gekennzeichnet, dass** das Gemisch von Lösungsmitteln ein Gemisch aus Wasser und Alkohol, Glykol oder Polyol 100/0 bis 0/100 ist.

8. Verwendung nach Anspruch 6, **dadurch gekennzeichnet, dass** das Lösungsmittel Wasser ist.

## Revendications

1. Utilisation d'au moins une substance stimulant l'expression d'un récepteur d'un neuromédiateur codé par le gène POMC choisi parmi MC-1R, MC-2R, et µ opioïde R, dans au moins un type de cellules de peau exprimant au moins un de ces récepteurs et un produit du gène POMC, comme ingrédient actif dans une composition cosmétique, dans laquelle ladite substance est un extrait de Millefeuille plante (*Achillea millefoium*), pour prévenir ou lutter contre les effets d'un stress induisant des variations observées au cours du vieillissement cutané, et/ou pour prévenir et/ou lutter contre la diminution de l'homéostasie épidermique dans le cadre général du vieillissement, et/ou pour améliorer la prolifération et la différenciation cellulaire, au niveau épidermique, et/ou pour prévenir et/ou lutter contre la diminution de l'épaisseur de l'épiderme au cours du vieillissement cutané chronobiologique ou photoinduit, ou pour générer une sensation de bien-être, et dans laquelle les cellules de la peau sont des kératinocytes.

2. Utilisation selon la revendication 1, dans laquelle ladite substance augmente ou maintient également l'expression de POMC.

3. Utilisation selon l'une quelconque des revendications 1 ou 2, pour stimuler l'expression dudit récepteur pour augmenter les effets cosmétiques de alpha-MSH et/ou ACTH et/ou β -endorphine.

4. Utilisation selon l'une quelconque des revendications précédentes, pour lutter contre ou prévenir une dérégulation des effets de alpha-MSH et/ou ACTH et/ou β -endorphine.

5. Utilisation selon l'une quelconque des revendications précédentes, **caractérisée en ce que** ladite substance est un extrait de plante, et est utilisée dans la composition cosmétique en une quantité comprise entre 0,01% et 5% (v/v).

6. Utilisation selon l'une quelconque des revendications précédentes, **caractérisée en ce que** ladite substance est un extrait de plante obtenu par macération de ladite plante à 1-10 % (p/p) dans un solvant ou un mélange de solvants.

7. Utilisation selon la revendication 6, **caractérisée en ce que** le mélange de solvants est un mélange d'eau et d'alcool, glycol ou polyol 100/0 à 0/100.

8. Utilisation selon la revendication 6, **caractérisée en ce que** ledit solvant est l'eau.
